# EUROPEAN PATENT APPLICATION

(11) **EP 2 308 373 A1**
(43) Date of publication of application: **13.04.2011**
(21) Application number: 09172781.8
(22) Date of filing: 12.10.2009
(51) Int. Cl.: A61B 5/103

(54) **Apparatus and method for analysing the gait of a person**

(71) Applicant: RSSCAN International NV, 2250 Olen (BE)
(72) Inventor: Wilssens, Jean-Pierre, B-9120, Beveren (BE)
(74) Representative: Bird, William Edward

(57) **Abstract**

The present invention relates to an apparatus for analysing the gait of a person, comprising: a treadmill (10) having a conveyor belt (12) guided over at least two rollers (16), wherein the surface side (18) of the conveyor belt (12) is a tread area, and having a frame (14) at least partly surrounding the conveyor belt (12); an encoder (20) for determining the speed of the conveyor belt (12); a pressure plate (18) located underneath the tread area of the conveyor belt (12) comprising an array of pressure sensors; at least one force sensor (26); electronic means (28) adapted to read out pressure distribution values of the array of pressure sensors and force values of the at least one force sensor (26); and analysing means (30) adapted to synchronise the pressure distribution values and the force values.

## Description

### FIELD OF THE INVENTION

The invention relates to an apparatus for analysing the gait of a person by measuring the pressure and the force exerted on a ground by the feet of a person who is walking or running.

### BACKGROUND INFORMATION

Repetitive movements such as jogging or running training can induce injuries. Running injuries commonly develop gradually, i.e. a cumulative effect of repetitive loading, however severe training sessions may also exacerbate an injury. A common injury is knee pain, however injuries of tendons such as the Achilles tendon or posterior tibial tendonitis, plantar fasciitis, pes planovalgum, metatarsalgia and calcaneal spurs, adductor strain, iliotibial band friction syndrome, iliotibial strain, tibial stress syndrome, patello-femoral pain, ankle sprain, back pain especially lower back pain and sciatica, hip or groin pain, thigh pain, foot sprain, and bursitis, have also been reported.

To alleviate these effects it has been proposed to correct training errors, correct muscle imbalance, identify and correct structural abnormalities, correct running style, use running shoes, identify injury early, treat the injury early, prescribe and use orthotics, etc. To achieve this a physical examination of the person is recommended including an identification and assessment of kinetic chain dysfunction as well as identification of previous injuries and measurement of anthropometric and functional characteristics associated with risk of running injury.

A wide variety of diagnostic methods are available to the doctor, e.g. CT-scan, X-ray, MRI scan as well as physical examination. However these are usually carried out in a static mode. There remains a need for a method of identifying potential injuries as well as to diagnose the cause of existing injuries which is easy to use, requires equipment which need not be in a clinic or hospital and which need not be operated by trained medical staff.

An apparatus is known from WO 95/31933 using a walking belt. During a constant walking phase pressure sensors are used to determine the dynamic pressure effect of the feet together with the change in relative position of selected parts of the body by two-dimensional angle measurements and a muscle potential measurement. Also the gait is monitored by video cameras.

US 4,813,436 describes another system for motion analysis comprising markers secured at various joints as well as pressure sensitive shoes or insoles. The subject is photographed during motion using two video carriers. The gait, the angular position of the various joints and other information indicative of walking characteristics are displayed.

US 6,010,465 describes an apparatus characterizing the gait of a person comprising a treadmill having a movable support surface, which is provided with multiple transducers mounted beneath the movable support surface. The person performs locomotion in the movable surface. The computer accepts a series of signals from each transducer and identifies the occurrence of heel-strike and toe-off and the person's activity as walking or running. The computer identifies two non-contiguous groups of transducers that are measuring a pressure greater than zero associated with the two feet and calculates quantities related to the pressure exerted by each foot during each phase of walking or running.

WO 2007/131542 describes a device for analyzing movement using a running belt, comprising a continuous belt guided over at least two track rollers, the surface of which serves as a running surface, a sensor system for determining a pressure distribution on a measuring plate located underneath the continuous belt, which has on the side facing the belt a plurality of pressure sensors arranged in the manner of a matrix, an analysis unit connected on the input side to the pressure sensors, which detects the position pressure distribution images of a moving vertebrate on the running belt and thereby detects the feeding motion thereof according to time and location, and a processing unit connected on the input side to the analysis unit, which, using the time and location-dependency of the pressure distribution images, generates measurement parameters to identify the movement of the vertrebrate, wherein a tachometer stage connected on the input side to the analysis unit is provided, which, using the time and location-dependency of the pressure distribution images, calculates an instantaneous speed of the continuous belt as an additional parameter of the analysis or processing unit.

These known apparatus require a complex and expensive assembly. Hence there is a need to provide equipment for analysing gait which is simple and unobtrusive. Furthermore, these apparatus only have the possibility of determining the pressure distribution that the feet exert on a floor, so that it is possible to determine the posture of the feet. But, these apparatus are not able to determine the force on the joints of the feet, with which it would be possible to determine what is being loaded on the feet and whether this is too much. But these two parameters are very important for sport medicine analyses.

Therefore, it is an object of the invention to provide an apparatus for analysing the gait of a person, which enables to determine the pressure distribution that the feet exert on a ground and the force on the joints of the feet without the need of complex equipment.

### SUMMARY OF THE INVENTION

The solution of the object is achieved by the features of claim 1. Preferred embodiments are given by the dependent claims.

The present invention includes an apparatus for analysing the gait of a person, comprising: a treadmill having a conveyor belt guided over at least two rollers, wherein the surface side of the conveyor belt is a tread area, and having a frame at least partly surrounding the conveyor belt; an encoder for determining the speed of the conveyor belt; a pressure plate located underneath the tread area of the conveyor belt comprising an array of pressure sensors; at least one force sensor; electronic means adapted to read out pressure distribution values of the array of pressure sensors and force values of the at least one force sensor; and analysing means adapted to synchronise the pressure distribution values and the force values.

With the apparatus according to the invention it is possible to measure pressure distribution values and force values. This is especially advantageous in sports medicine applications since it is possible to determine the posture, that means the positions of the bones, muscles, ligaments etc, and it is possible to determine the force on the joints of a walking or running person. The posture is governed by the feet of the person, especially how the feet are placed on a ground during walking or running. This determines the ankle and the orientation of the feet places on the ground. Thus, the posture may be determined by the pressure distribution that the feet exert on a ground. The pressure distribution is measured by an array of pressure sensors arranged at a pressure plate underneath a tread area of a conveyor belt of a treadmill. By additionally measuring the forces on the joints of the feet it is possible to determine the amount of the load to the feet to be able to estimate if his load is too much, so that injuries caused by defective position of the feet may occur. The force is measured by at least one force sensor which is positioned at the treadmill independent of the pressure sensors.

The pressure plate comprising an array of pressure sensors to measure the pressure distribution is placed underneath the conveyor belt, so that the pressure distribution may be measured while a person is training on the treadmill. The pressure plate is arranged at a fixed position at the treadmill, so that the conveyor belt may move over the stationary pressure plate. It is possible to apply a layer of Teflon on the pressure plate to make the conveyor belt slip easier over the pressure plate. In order to know exactly how the pressure distribution is related to the feet it is necessary to know the speed of the conveyor belt. As the conveyor belt moves towards the back of the treadmill it takes the feet with it, so that the position of the feet changes with the time. Thus, an encoder is provided to determine the speed of the conveyor belt to eliminate the movement of the feet by determining the gait of the person. To read out the pressure distribution values of the array of pressure sensors and the force values of the at least one force sensor electronic means are provided. To obtain results, which are instantly usable, analysing means are provided, with which it is possible to synchronise the pressure distribution values and the force values with each other. Thus, it is possible to determine the pressure distribution and the force to a defined point of time. Hence, it is possible to determine the relevant pressure distribution at a moment T and to determine the forces at the moment T. This facilitates the diagnosis possibilities in sports medicine applications. Preferably, the pressure distribution values and the force values are synchronised with a time frame. Such a time frame may be the output of the encoder or it is possible to use an either internal or external independent clock.

According to a preferred embodiment of the invention the encoder is connected with a motor driving the rollers. This enables a very simple measuring of the speed of the conveyor belt, since the encoder is directly connected with the motor of the rollers. The encoder on the motor measures the speed of the conveyor belt. The measured speed is transmitted to the electronic means to be able to eliminate the movement of the conveyor belt out of the measured pressure values and the force values to obtain pressure distribution values and force values for each foot as if the person was running on a spot. Instead of positioning the encoder at the motor, it may be possible to measure the speed of the conveyor belt by marks on the conveyor belt or it may be possible to use a roller to measure the speed of the conveyor belt. But these methods are more complex than measuring the speed directly at the motor, since additional equipment is needed. Using an encoder directly connected to the motor has furthermore the advantage that standard device may be used and a treadmill may easily be refitted by these standard devices.

To obtain a very precisely measuring of the forces on the joints, the at least one force sensor is adapted to measure forces in three coordinate directions X, Y and Z according to a further preferred embodiment of the invention.

There are preferably two possible embodiments of positioning the at least one force sensor at the treadmill.

According to a first possible embodiment of the invention the pressure plate is connected to the frame of the treadmill, wherein the at least one force sensor is positioned at the frame of the treadmill. Since the pressure plate is connected to the frame of the treadmill all forces applied to the conveyor belt are transmitted through the pressure plate to the frame. To be independent from the pressure sensors the force sensors may be positioned at the frame or the feet on which the frame stands, so that the total forces may be measured.

Preferably, four force sensors are provided, wherein the frame provides four corners and each corner of the frame is provided with one force sensor. Each force sensor may measure forces in the three coordinate directions X, Y and Z. The measured values of these four force sensor are synchronised with each other, so that very exactly force values may be obtained. It is especially advantageous to provide one force sensor in each corner of the frame if the conveyor belt is inclinable, since in an inclined position there will be more force on the back of the treadmill. To avoid falsified measured values in this inclined position it is advantageous to locate multiple force sensors around the conveyor belt.

According to a second possible embodiment of the invention the pressure plate provides at least two legs adapted to position the pressure plate on a ground, wherein at least one force sensor is positioned in one of the at least two legs. Thus, the pressure plate is independent from the frame of the treadmill and is not fixed to the frame. This has the advantage that vibrations of the motor are not transmitted to the force sensor. Thus the force values of the force sensor are not falsified by the vibrations of the motor driving the rollers of the treadmill. To be independent from the pressure sensors in the pressure plate the force sensors are positioned in the legs of the pressure plate.

It is further preferred to provide a three dimensional imaging system adapted to capture a three dimensional image, especially a three dimensional video image, of the person using the treadmill, wherein the three dimensional imaging system is connected to the electronic means. The person using the treadmill may be filmed with the three dimensional imaging system to capture a three dimensional image of the person to be able to analyse the feet position very exactly. The data of the three dimensional imaging system is read out by the electronic means to obtain a three dimensional image, which may be shown on a display or screen. If the pressure distribution values and the force values are also shown on a screen, it is possible to view the running person in a three dimensional image while being able to study the pressure values as pressure plots and the force values as force dynamics. This facilitates to correct gait and/or posture problems. By the three dimensional imaging system it is possible to depict different feet positions in different angles without the need of special clothing or equipment which the person had to wear.

Preferably, the three dimensional imaging system comprises structured light appliance or a three dimensional stereoscopic camera system. Structured light is the projection of a light pattern at a known angle onto the person, which is very useful for imaging and acquiring dimensional information of the person, especially the feet of the person. The structured light appliance for acquiring a three dimensional image of a scene preferably comprises at least one pattern generator for projecting pattern on the scene, at least one camera for making an image or images of the scene, and computing means for determining the shape or image of the scene. Especially the structured light appliance for obtaining a three dimensional shape of an image of a free-form object may comprise at least one pattern generator for simultaneously projecting a pattern of components having a known relative positioning, at least one imaging device for acquiring an image of the pattern projected onto the free-form object, and means for selecting one component out of the acquired image as a reference component, means for determining relative positional coordinates of the pattern components other than the reference component in the acquired image with reference to the reference component, and computing means for determining relative depths of positions on said three dimensional shape of the free-form object by linear transformations of the relative positional coordinates based on one of a pseudo-orthographic and an orthographic geometrical model. The image may be acquired via relative positions of components in the pattern, for instance by determining the relative sequence of pattern lines. Preferably a single image will suffice to acquire a three dimensional image description and an acquisition time is required which is no longer than that for making a single image. Since the recording time of a single image may remain limited and since reconstructions may be preformed for a quick succession of different images, moving objects and their three dimensional movements and changes of shape may be reconstructed.

According to a further embodiment of the invention transmission means are provided adapted to send a timing signal from the encoder to the electronic means. The timing signal allows to step through or index through the measurements of the pressure distribution and the force and through the three dimensional images. Thus, it is possible to create frames of the pressure distribution, frames of the forces and frames of the three dimensional images from the same moment in time, so that it is possible to see each displayed at the same time on one or more displays and afterwards it is possible to increment to the next time position. To do this a timing signal may be sent from the encoder to the read out electronic means of the pressure sensors, the force sensors and the three dimensional imaging system. In this way for example a physician may choose small time steps and see how the pressure distribution and the force distribution change while being able to relate this to the three dimensional imaging system in order to see due to which movements and postures of the feet the specific pressure or force distributions may occur.

### BRIEF DESCRIPTION OF THE DRAWING

These and other aspects of the invention will be apparent from and elucidated with reference to the embodiment described hereinafter.

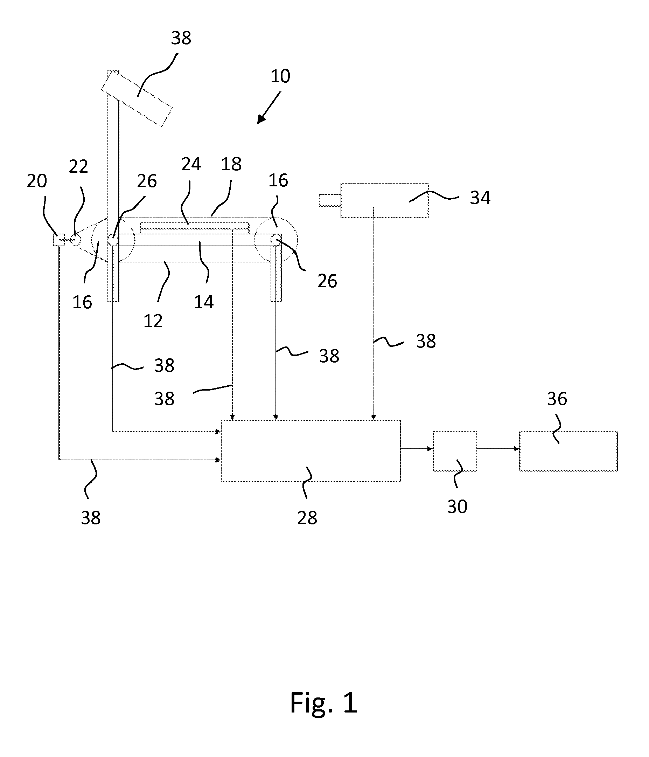

Fig. 1 is a schematic representation of an apparatus for analysing the gait of a person in accordance with an embodiment of the present invention.

### DESCRIPTION OF THE ILLUSTRATIVE EMBODIMENT

The present invention relates to an apparatus for diagnosis of problems of body posture or movement especially of stance and gait defects or problems as well as for diagnosing strains caused by repetitive movement during training. For example, the present invention uses an apparatus for displaying pressures and forces on the feet and relating these to movements of the human or animal body, especially movements of the lower skeleton, e.g. foot, ankle, knee or hip. In particular means are provided for display of eversion or inversion motion of the foot. The methods of the present invention do not require the use of a video camera for every human or animal to be examined but only for capturing initial values.

The present invention provides means for the measurement of force and pressure distribution caused by the foot of a person or animal especially when walking, hopping, jumping or running and the derivation from these measurements of information useful in the diagnosis or prevention or remediation of injury. In particular the information which is to be derived is movement of parts of the kinematic chain attached to the foot, especially rotation of the tibia, that is either external or internal rotation.

The present invention is based on the observation that although the limbs with their joints theoretically have a large number of degrees of freedom and a variety of movements, in fact these movements are limited when the person or animal is running, hopping, jumping and walking by the need to keep the body in balance. Also the parts of the kinematic chain attached to the foot are not independent of each other. For instance when a human is standing upright and rotates the upper body, the pressure distribution of the feet changes caused by the internal linkages between the parts of the kinematic chain between the upper body and the foot. The present invention makes use of these dependencies to display motion of parts of the kinematic chain based pressure measurements and force measurements carried out on the foot or feet.

In accordance with embodiments of the present invention the kinematics of parts of the lower limb such as the foot and also the ankle, tibia etc. are displayed in a three dimensional imaging system based on measurements of the temporal evolution of local pressures underneath the foot, e.g. during hopping, jumping, walking or running over a pressure plate. In one embodiment the kinematic modelling is provided by determining relationships empirically between the temporal evolution of the local pressures and the movement of the bones of the foot and the bones of the kinematic chain attached to the foot.

Fig. 1 shows a schematic view of an apparatus according to the invention for analysing the gait of a person. The apparatus comprises a treadmill 10 having a conveyor belt 12 and a frame 14 at least partly surrounding the conveyor belt 12. The conveyor belt 12 is guided over two rollers 16, wherein the surface side 18 is a tread area for the person running or walking on the conveyor belt 12. To determine the speed of the conveyor belt 12 an encoder 20 is provided, which is connected with a motor 22 driving the rollers. Thus, the speed of the conveyor belt 12 may be directly measured and it is simple to refit a treadmill 10 with such an encoder 20. Underneath the surface side 18 of the conveyor belt 12 forming the tread area a pressure plate 24 is located comprising an array of pressure sensors, especially arranged in a regular two dimensional array (not shown). The pressure sensors are preferably resistive sensors but may also be capacitive or inductive sensors. Connections are provided for measuring the resistance of each individual sensor and outputting this as a signal. Resistive sensors generally have a nonlinear force/resistance characteristic. There may be several thousand resistive sensors arranged in an array of rows and columns or a similar organised structure and the sensors are preferably distributed evenly over the pressure plate 18. The position of each sensor on the pressure plate 18 is preferably known and this position is stored in electronic means 28 and associated with the specific sensor. The sensors may be formed from a single sheet of resistive material and covered by a suitable outer thin flexible layer (not shown). Underneath the layer, a substantially rigid plate may be provided which may be several millimetres thick and made of a suitable rigid material such as steel or rigid plastic.

The pressure plate 24 is connected to the rectangular shaped frame 14 of the treadmill 10. Additionally to the array of pressure sensors four force sensors 26 are provided, wherein one of these force sensors 26 is positioned at one corner of the frame 14 of the treadmill 10, respectively. Thus, it is possible to measure the pressure distribution that the feet exert on a ground to be able to determine the posture of the feet and moreover it is possible to measure the forces on the joints of the feet to be able to determine what is being loaded on the joints to evaluate if this load is too much for the joints. To determine these values electronic means 28 are provided to read out the pressure distribution values measured by the pressure sensors and to read out the force values measured by the force sensors 26. To obtain results, which are instantly usable, analysing means 30 are provided, with which it is possible to synchronise the pressure distribution values and the force values from the electronic means 28 with each other. Thus, it is possible to determine the pressure distribution and the force to a defined point of time. Hence, it is possible to determine the relevant pressure distribution at a moment T and to determine the forces at the moment T. This facilitates the diagnosis possibilities in sports medicine applications. Preferably, the pressure distribution values and the force values are synchronised with a time frame. This time frame is obtained by an output of the encoder 20 read out by the electronic means 32, in which transmission means 38 are provided adapted to send a timing signal from the encoder 20 to the electronic means 28. Transmission means 38 are also provided between the pressure plate 18, the force sensors 26, the encoder 20, the three dimensional imaging system 34 and the electronic means 28 to link the electronic means 28 with these parts of the inventive apparatus. Transmission means 38 may include cables, optical, e.g. infrared, microwave transmission devices or similar, i.e. the present invention includes both a direct connection between pressure plate 18, force sensors 26, the encoder 20 and the three dimensional imaging system 34 with the electronic means 28 via cables as well as remote connection via any form of electromagnetic radiation.

To obtain a more a detailed analyses of the gait of a person walking or running on the treadmill 10 any system of motion tracking or motion capture can be used with the present invention to capture a three dimensional image of the person using the treadmill 10. The data of the motion capture system is read out by the electronic means and analysed by analysing means to obtain a three dimensional image for example in form of a video, which may be shown on a display. For example a three dimensional imaging system 34, optionally a three dimensional video camera, is provided adapted to capture a three dimensional image of the person using the treadmill 10. The data of the three dimensional imaging system 34 is read out by the electronic means 28 and analysed by the analysing means 30 to obtain a three dimensional image for example in form of a video, which may be shown on a display 36. The pressure distribution values and the force values are also shown on a display 36, preferably each value and the image is shown on an own display 36. Thus, it is possible to compare the pressure distribution, the force and the image with each other to obtain a very precise analysis of the gait of a person.

Other methods of motion capture may be used. For example the user of the treadmill can wear markers near each joint to identify the motion by the positions or angles between the markers. Acoustic, inertial, LED, magnetic or reflective markers, or combinations of any of these, can be used and tracked. The frequency rate for capturing the motion is preferably at least twice the frequency of the desired motion, to submillimeter positions. Optical systems can be used that analyse data captured from image sensors or cameras to triangulate the 3D position of each part of the user. Special markers can be attached to the user or surface features are identified dynamically for each particular user and tracked. For example passive infra-red systems may be used whereby the markers reflect infra-red light. These systems produce data with 3 degrees of freedom for each marker, and rotational information must be inferred from the relative orientation of three or more markers; for instance hip, elbow, knee and ankle provide the angle of the knee.

Passive optical systems can be used in which markers reflect light back that is generated near the cameras lens using a light source, e.g. an infra-red source. The camera's threshold can be adjusted so only the bright reflective markers will be sampled, ignoring skin and fabric or alternatively filters may be used that only allow the infra-red light through. A reference object with markers attached at known positions can be used to calibrate the cameras and obtain their positions.

Active optical systems can also be used to triangulate positions by using active light sources such as LED's, e.g. active infra-red light emitting sources can be used. Rather than reflecting light back that is generated externally, the markers themselves are powered to emit their own light. Active marker systems can further be refined by strobing each marker, e.g. modulating the amplitude or pulse width to provide a unique identification of each marker.

Moreover, to check the treadmill 10, especially the conveyor belt 12, for proper operation a control unit 38 is provided, which is attached to the frame 14 above the tread area 18 of the conveyor belt 12.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. Apparatus for analysing the gait of a person, comprising:
- a treadmill (10) having a conveyor belt (12) guided over at least two rollers (16), wherein the surface side (18) of the conveyor belt (12) is a tread area, and having a frame (14) at least partly surrounding the conveyor belt (12);
- an encoder (20) for determining the speed of the conveyor belt (12);
- a pressure plate (18) located underneath the tread area of the conveyor belt (12) comprising an array of pressure sensors;
- at least one force sensor (26);
- electronic means (28) adapted to read out pressure distribution values of the array of pressure sensors and force values of the at least one force sensor (26); and
- analysing means (30) adapted to synchronise the pressure distribution values and the force values.

2. Apparatus according to claim 1, wherein the encoder (20) is connected with a motor (22) driving the rollers (16).

3. Apparatus according to anyone of the preceding claims, wherein the at least one force sensor (26) is adapted to measure forces in three coordinate directions.

4. Apparatus according to anyone of the preceding claims, wherein the pressure plate (18) is connected to the frame (14) of the treadmill (10), wherein the at least one force sensor (26) is positioned at the frame (14) of the treadmill (10).

5. Apparatus according to anyone of the preceding claims, wherein four force sensors (26) are provided, wherein the frame (14) provides four corners and each corner of the frame (14) is provided with one force sensor (26).

6. Apparatus according to anyone of the preceding claims, wherein the pressure plate (18) provides at least two legs adapted to position the pressure plate (18) on a ground, wherein the at least one force sensor (26) is positioned in one of the at least two legs.

7. Apparatus according to anyone of the preceding claims, wherein a three dimensional motion capture system is provided adapted to capture a motion of parts of the person using the treadmill (10), wherein the motion capture system is connected to the electronic means (28).

8. Apparatus according to anyone of the preceding claims, wherein a three dimensional imaging system (34) is provided adapted to capture a three dimensional image of the person using the treadmill (10), wherein the three dimensional imaging system (34) is connected to the electronic means (28).

9. Apparatus according to claim 8, wherein the three dimensional imaging system (34) comprises structured light appliance or a three dimensional stereoscopic camera system.

10. Apparatus according to anyone of the preceding claims, wherein transmission means are provided adapted to send a timing signal from the encoder (20) to the electronic means (28).
